# EUROPÄISCHE PATENTSCHRIFT

(11) **EP 0 934 079 B1**
(45) Veröffentlichungstag und Bekanntmachung des Hinweises auf die Patenterteilung: **20.02.2002**
(21) Anmeldenummer: 97943820.7
(22) Anmeldetag: 01.09.1997
(51) Int. Cl.: A61K 47/18

(54) **PHARMAZEUTISCHE ZUSAMMENSETZUNG ENTHALTEND LORNOXICAM UND EIN DINATRIUMSALZ DER EDTA**
PHARMACEUTICAL COMPOSITION CONTAINING LORNOXICAM AND A DISODIC SALT OF EDTA
COMPOSITION PHARMACEUTIQUE CONTENANT DU LORNOXICAM ET UN SEL DISODIQUE DE L'ACIDE ETHYLENEDIAMINE TETRACETIQUE

(30) Priorität: 03.09.1996 AT 156496
(43) Veröffentlichungstag der Anmeldung: 11.08.1999
(73) Patentinhaber: Nycomed Austria GmbH, 4021 Linz (AT)
(72) Erfinder: RUCKENDORFER, Hermann, A-4193 Reichenthal 170 (AT); EGGER, Philippus, A-4210 Gallneukirchen (AT)
(74) Vertreter: Landgraf, Elvira, Dipl.-Ing.
(86) Internationale Anmeldenummer: EP9704742
(87) Internationale Veröffentlichungsnummer: WO9809654

(56) Entgegenhaltungen:
- WO-A-96/41646
- BE-A- 899 587
- US-A- 5 362 758

## Beschreibung

Die Erfindung betrifft eine klare pharmazeutische Zusammensetzung für die parenterale Applikation enthaltend 6-Chlor-4-hydroxy-2-methyl-N-2-pyridyl-2H-thieno-[2,3-e]- 1,2-thiazin-carboxamide-1,1-dioxide (Lornoxicam) oder ein pharmazeutisch verträgliches Salz davon.

Lornoxicam ist ein nicht steroidaler antiinflammatorischer Wirkstoff und kann zur Behandlung von akuten und chronischen Schmerzzuständen, die mit entzündlichen Prozessen einhergehen, z.B. postoperativern Schmerz, Arthritis, Rheumatismus, Weichteilverletzungen u. dgl. eingesetzt werden.

Nicht steroidale antiinflammatorische Wirkstoffe (NSAIDs) sind im allgemeinen sehr schwer, teilweise nahezu unlöslich in Wasser. Diese Unlöslichkeit bereitet schwerwiegende Problem bei der Formulierung derartiger Verbindungen zu Lösungen für die parenterale oder ophthalmische Applikation, insbesondere für die intravenöse Anwendung.
Die Erhöhung der Löslichkeit kann beispielsweise durch Salzbildung oder Komplexierung des Wirkstoffes erfolgen.

Lomoxicam ist ein äußerst schwer lösliches NSAID, das zudem in flüssiger Formulierung instabil ist, wobei durch Oxidation und hydrolytische Spaltung zahlreiche Nebenprodukte entstehen. Die Stabilität der flüssigen Formulierung kann durch Lyophylisieren erhöht werden, die aus dem Lyophylisat erhaltenen flüssigen Formulierungen weisen aber eine nicht unerhebliche Trübung auf. Dadurch sind die so hergestellten parenteralen oder ophthalmischen Formulierungen nicht brauchbar.

Die Verwendung von EDTA und ihren Salzen zur Stabilisierung von Lösungen von pharmazeutisch anwendbaren Verbindungen sind bekannt. Sie werden insbesondere zur Komplexierung von Metallionen, wie Kupfer-, Eisen-, Manganionen, die die Oxidation der pharmazeutischen Wirkstoffs hervorrufen oder beschleunigen, verwendet.

Überraschenderweise konnte nun gefunden werden, dass der Zusatz von geringen Mengen des Dinatriumsalzes von EDTA die Trübung von Lösungen aus lyophylisiertem Lornoxicam verhindert. Dieser Effekt trat auf ohne Einfluss auf die chemische Stabilität des Wirkstoffs gegenüber Oxidationsreaktionen zu haben, wie eine vergleichende Stabilitätsuntersuchung über 3 Jahre an Lornoxicam-Lyophylisatformulierungen mit und ohne Dinatrium-EDTA ergab. Im Gegensatz zum Dinatriumsalz der EDTA zeigte das Calciumnatriumsalz von EDTA keinen Einfluss auf die Trübung der Lornoxicam-Formulierung.

Gegenstand der Erfindung sind daher pharmazeutische Zusammensetzungen für die parenterale oder ophthalmische Applikation enthaltend Lornoxicam oder ein pharmazeutisch verträgliches Salz davon und ein Dinatriumsalz der EDTA, mit der Maßgabe, dass die pharmazeutische Formulierung kein Cyclodextrin aus der Gruppe alpha cyclodextrin sowie der hydroxypropylierten oder sulphoalkylierten Derivate von alpha-oder beta- Cyclodextrin enthält.

Gemäß der vorliegenden Erfindung wird das Dinatriumsalz der EDTA dazu eingesetzt, die auftretende Trübung von Lösungen, die lyophylisierte Lornoxicam enthalten, zu verhindern.
Andere Methoden, beispielsweise Filtrieren der Lösung, Zusatz von Ascorbinsäure oder Natriumbisulfit oder Zusatz eines Komplexierungsmittels wie Polyvidone 17 PF konnten die Trübung der Lösung nicht verhindern.

Ein Lösen in organischen Lösungsmitteln oder deren Mischungen, wie beispielsweise Propylenglykol, Polyethylenglykol u. dgl. reduziert zwar die Trübung der Lösungen, kommt aber wegen der bei der Injektion derartiger Lösungen auftretenden Schmerzen durch Hyperosmolalität nicht in Frage.
Das Dinatriumsalz der EDTA wird dabei nur in sehr geringer Menge zugesetzt, etwa 0,1 - 5 mg pro 20 ml Lösung, vorzugsweise 0,25 - 4 mg pro 10 ml Lösung.

Die so hergestellten Lösungen weisen Trübungswerte von 3- 12 auf und sind daher als klare Lösungen nach Ph. Eur. zu klassifizieren und für die parenterale und ophthalmische Anwendung geeignet.

### Beispiel 1

Standardformulierung

| | |
|---|---|
| Lornoxicam | 8 mg |
| Mannitol | 100 mg |
| Trometamol | 2 mg |

gelöst in 2 ml Wasser ad inj.
Die Klarheit der Lösung wurde auf folgende Weise bestimmt:
a) Ph.Eur.-Verfahren
   Die Probe wird in diffusem Tageslicht mit Wasser oder einer Referenzsubstanz gegen einen schwarzen Hintergrund verglichen.
b) Trübungsmessung
   Die Probe wird in einen monochromatischen Lichtstrahl bei einer Wellenlänge von 564 nm gemessen, wobei als Referenz eine Glasküvette mit standardisierter Trübung dient.
   Die Standardformulierung in rekonstituierter Lösung wies Trübungswerte zwischen 140 und 200 auf, wobei nur Lösungen, die Trübungswerte unter 70 - 100 aufweisen nach Ph. Eur. als klar klassifiziert werden können.

### Beispiel 2

| | |
|---|---|
| Lornoxicam | 8 mg |
| Mannitol | 100 mg |
| Trometamol | 12 mg |
| EDTA-Dinatrium | 0,05 mg - 0,8 mg |

gelöst in 2 ml Wasser ad inj.
Die Lösungen dieses Typs von LornoxicamLyophylisat-Rezeptur wiesen Trübungswerte von 3 -12 in der rekonstituierten Lösung auf.

### Beispiel 3:

| | |
|---|---|
| Lornoxicam | 8 mg |
| Mannitol | 100 mg |
| Trometamol | 12 mg |
| EDTA-Dinatrium Calcium | 0,05 mg - 0,8 mg |

Die Lösungen dieses Typs von Lornoxicam Lyophilisat-Rezeptur wiesen Trübungswerte von 160 - 340 in der rekonstituierten Lösung auf.

### Beispiel 4:

Vergleichende Stabilitätsdaten nach 36 Monaten Lagerung mit Lomoxicam Lyophilisat-Formulierungen hergestellt nach den Grundrezepturen in Beispiel 1 (ohne EDTA-Dinatrium) und Beispiel 2 (mit EDTA-Dinatrium).

| Testparameter | Formulierung ohne EDTA-Dinatrium* | Formulierung mit EDTA-Dinatrium** |
|---|---|---|
| Wirkstoffgehalt | 8,44 mg | 8,44 mg |
| Oxidationsprodukte: | | |
| Verbindung A | ∠ 0,1 % | 0,1 % |
| Verbindung B | 0,2 % | 0,2 % |

| | | |
|---|---|---|
| * Lornoxicam 8 mg; Mannitol 100 mg; Trometamol 12 mg | | |
| ** Lornoxicam 8 mg; Mannitol 100 mg; Trometamol 12 mg; EDTA-Dinatrium 0,2 mg Verbindung A: N-(2-Aminopyridyl)oxalsäure Verbindung B: 5-Chlor-3-sulphinothiophen-2-carbonsäure | | |

## Patentansprüche

1. Pharmazeutische Zusammensetzungen für die parenterale oder ophthalmische Applikation enthaltend Lornoxicam oder ein pharmazeutisch verträgliches Salz davon und ein Dinatriumsalz der EDTA, mit der Maßgabe, dass die pharmazeutische Formulierung kein Cyclodextrin aus der Gruppe alpha-Cyclodextrin, Sulphobutyl-alpha-Cyclodextrin, Hydroxypropyl-alpha-Cyclodextrin, Sulphobutyl- beta-Cyclodextrin und 2-Hydroxypropyl-beta-Cyclodextrin enthält.

2. Pharmazeutische Zusammensetzung gemäß Anspruch 1, **dadurch gekennzeichnet, dass** das Dinatriumsalz der EDTA in einer Konzentration von 0,1 - 5 mg pro 10 ml Lösung eingesetzt wird.

3. Pharmazeutische Zusammensetzung nach Anspruch 1, **dadurch gekennzeichnet, dass** das Dinatriumsalz der EDTA in einer Konzentration von 0,25 - 4 mg pro 10 ml Lösung eingesetzt wird.

## Claims

1. Pharmaceutical compositions for parenteral or ophthalmic administration containing lornoxicam or a pharmaceutically acceptable salt thereof and a disodium salt of EDTA, with the proviso that the pharmaceutical formulation does not contain a cyclodextrin from the group comprising alpha-cyclodextrin, sulfobutyl-alpha-cyclodextrin, hydroxypropyl-alpha-cyclodextrin, sulfobutyl-betacyclodextrin and 2-hydroxypropyl-beta-cyclodextrin.

2. Pharmaceutical composition according to Claim 1, **characterized in that** the disodium salt of EDTA is used in a concentration of 0.1 - 5 mg per 10 ml of solution.

3. Pharmaceutical composition according to Claim 1, **characterized in that** the disodium salt of EDTA is used in a concentration of 0.25 - 4 mg per 10 ml of solution.

## Revendications

1. Composition pharmaceutique pour une application parentérale ou ophtalmique, contenant du lornoxicam ou un sel compatible du point de vue pharmaceutique, de ce dernier et un sel disodique de l'EDTA, avec la condition selon laquelle la formulation pharmaceutique ne contient pas de cyclodextrine du groupe alpha-cyclodextrine, sulfbutyl-alpha-cyclodextrile, hydroxypropyl-alpha-cyclodextrine, sulfobutyl-bêta-cyclo-dextrine et 2-hydroxypropyle-béta-cyclodextrine.

2. Composition pharmaceutique selon la revendication 1, **caractérisée en ce que** le sel disodique de l'EDTA est utilisé en une concentration de 0,1-5 mg pour 10 ml de solution.

3. Composition pharmaceutique selon la revendication 1, **caractérisée en ce que** le sel disodique de l'EDTA est utilisable en une concentration de 0,25-4 mg pour 10 ml de solution.
